# EUROPEAN PATENT APPLICATION

(11) **EP 4 682 135 A1**
(43) Date of publication of application: **21.01.2026**
(21) Application number: 24382775.5
(22) Date of filing: 17.07.2024
(51) Int. Cl.: C05F 17/05, C05F 17/60, C05F 17/90, C05F 17/907, C05F 17/914

(54) **ORGANIC WASTE PROCESSING AND LARVAE FATTENING MACHINE**

(71) Applicant: Innolarva, SCCL, 08302 Mataró Barcelona (ES)
(72) Inventor: Genovese, Pablo Alberto, 08302 MATARÓ (ES); Reyes Fernandez, Oscar, 08302 MATARÓ (ES)
(74) Representative: Pons IP

(57) **Abstract**

The invention relates to a machine for organic waste processing and larvae fattening that comprises a structure (10) which holds:
- a drum (1), configured to contain the organic waste and the larvae,
- blades (3), located within the drum (1), so that the contain is shredded and not only moved,
- a handwheel (5) connected to the drum (1) through a gearbox (6), to empty the contain of the drum (1),
- a first motor (2) for the rotation of the drum (1),
- an electrical panel (9), to manage the machine,
- a lid (7) to close the opening of the drum (1),
- a second motor (4) for the rotation of the blades (3), and
- a heating blanket (8) located around the drum (1) with a thermostat,
wherein
- the second motor (4) rotates slower than the drum (1) and in the opposite direction.

## Description

### OBJECT OF THE INVENTION

The following invention describes a machine designed for the disposal of organic waste through the use of insect larvae. The machine is equipped with mechanical systems that enable the larvae to digest biowaste effectively. It comprises a structure, two motors, a gearbox, a heating and temperature regulation system, a ventilation system and a shredding system with blades.

The machine enables the shredding of organic waste and provides optimal conditions for larvae fattening during waste processing. It is also suitable for shredding wet materials without the need for added water.

### BACKGROUND OF THE INVENTION

An extensive search is conducted to identify any similarities with other types of devices, in order to offer a more efficient solution to the issue of organic waste management.

Document ES2331452B2 presents a system for eliminating organic waste using insect larvae. The equipment comprises a digester that includes a digestion chamber delimited by a separating mesh. The waste is fed through the top of the device, forming a layer of dry compound on the screen. However, the effectiveness of this invention is questioned due to the lack of movement affecting the aeration of the compost. Although the text mentions the possibility of forced aeration, it does not specify any temperature control. This lack of specification could negatively impact the optimal development of the larvae, especially Hermetia Illucens, used in the process. Therefore, the machine in the current application is an improvement over the equipment mentioned above.

The system described in ES2418437T3 processes organic waste using insect larvae in flat reaction vessels stacked in a block. Each reaction vessel contains organic waste and is separated by air gaps. The block is surrounded by a plant enclosure with at least one wall adjacent to the vessels and openings to allow air circulation. A feeder system loads the waste, preferably in the form of an elongated belt suspended between rollers. The enclosed drawings illustrate the arrangement and function of the system, featuring versions with columnar and row processing blocks.

Document ES2793482T3 presents a method for the bioconversion and biostabilization of organic and municipal waste using insect larvae. The proposed bioreactor enables the accumulation of compost produced by waste treatment. The process involves distributing waste in the bioreactor and introducing larvae or newly hatched eggs. The larvae feed on the waste and migrate to dry areas at the end of their development cycle. An automated collection system is used to collect the larvae and transport them for further processing. This method provides a solution for treating various types of organic waste and can be integrated into controlled food chains to improve efficiency in waste management.

Document US6001146A describes a device designed to treat putrescible waste using fly larvae. The device consists of a conveyor belt with areas for waste reception, treatment, and evacuation, as well as means for distributing the waste and depositing fly larvae or eggs. The larvae and waste are removed from the belt using graded light or heat, taking advantage of the larvae's negative phototropy and sensitivity to heat.

The process creates an odorless compost, and the larvae, which are rich in protein and chitin, are collected for commercial use. This method involves waste distribution, larval deposition, hatching, and subsequent removal of waste and larvae from the belt. Temperature and humidity are controlled to maximize larval growth in a closed, controlled environment.

Document US2022030656547A1 describes a conveyor belt with specific zones for receiving, treating, and disposing of waste. The purpose of this conveyor belt is to mix insects and plant biomass, which are the primary components of the biomass composition. Additionally, the machine is equipped with a corrugated roller mill that grinds the grains to process the mixture of insects and plant biomass. The mill has a feeder that directs the mixture into it and grinds it to obtain the desired biomass composition. The design of the machine is noteworthy for its ability to facilitate efficient waste management in industrial insect farming and produce useful biomass for various applications. This overcomes regulatory and economic issues associated with insect waste management.

### DESCRIPTION OF THE INVENTION

For the sake of clarity, following are some definitions of the terms used in the current application.

The term 'digester' refers to a machine or device that enables the recycling of organic waste through biological synthesis, typically using bacteria or larvae, to process the waste via the digestion process.

The term 'bio-waste' refers to waste from food or agricultural processes that involve organic waste. Bio-waste is treated and used to develop slurry, fertilizers, compost, animal feed, and other products.

The term 'larvae' refers to the embryonic stage of insects, which undergo metamorphosis to reach adulthood. Their primary function is to accumulate energy for further development.

The term 'fattening' refers to the growth process of the larvae. During this process, they digest organic waste and increase in mass.

The term 'machine' is defined as a collection of mobile or fixed components that operate to harness, direct, or transform energy, or to perform work for a specific purpose.

The term 'biomass' refers to plant matter used for energy transformation and applications in the primary sector.

The invention relates to a machine for the organic waste processing and larvae fattening. The machine comprises a structure which holds:
- a drum, configured to contain the organic waste and the larvae,
- a lid to close the opening of the drum,
- blades located within the drum so that the contain is shredded and not only moved,
- a handwheel connected to the drum through a gearbox, to empty the contain of the drum,
- a first motor for the rotation of the drum,
- a second motor for the rotation of the blades,
- an electrical panel, to manage the machine, and
- a heating blanket located around the drum.

In order to have optimal conditions for the digestive process of the larvae, the heating blanket comprises a thermostat to regulate the temperature within the drum. The thermostat controls the temperature within the drum in a determined range which varies between 24 and 35 °C.

Besides, the second motor rotates at a slower angular speed than the drum and in the opposite direction, in order to accelerate the shredding process.

The drum may also comprise holes uniformly separated around the drum. Each hole comprises a corresponding plug so that the holes are normally closed and may be opened in order to evacuate water generated during the shredding process. In order to easily evacuate the water, the holes are located in the corner of the drum at the opposite side of the opening.

The structure of the machine may also comprise wheels, so that it can be easily moved.

The invention also relates to a procedure for the processing of organic waste and the fattening of larvae with the machine above described.

This procedure comprises the following steps, starting with the machine off:
a) open the lid,
b) load the drum with organic waste,
c) close the lid,
d) switch on the first motor and the second motor,
e) let the machine work for a predetermined period of time,
f) switch off the motors,
g) open the lid,
h) introduce the larvae inside the drum,
i) close the lid,
j) switch on the heating blanket,
k) let the digestion process work for a predetermined period of time,
l) switch off the heating blanket,
m) switch on the first motor for a predetermined period of time,
n) decide whether to go to step j) or o)
o) turn off the heating blanket,
p) open the lid,
q) turn the handwheel to empty the drum.

The decision in step n) depends on the status of the digestive process. If the process is considered to be far to be completed and may be more accomplished, the option to return to step j) is considered.

Step m) takes place at least once a day in order to aerate the volume of the drum and not to let the larvae get crushed.

The procedure may also comprise the following steps after step o):
o1) switch on the first motor and the second motor,
o2) let the machine work for a predetermined period of time,
o3) switch off the motors.

Additionally, the procedure may also comprise the step of opening the plurality of holes.

### DESCRIPTION OF THE DRAWINGS

For a better understanding of the present invention, the figures listed below are included in this specification.

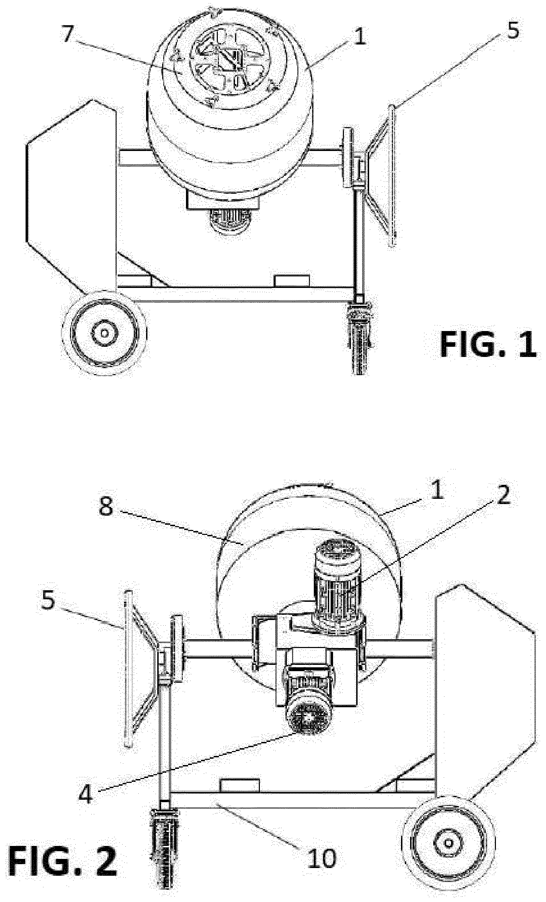

Figure 1 displays a front view of the machine of the invention.
Figure 2 displays a rear view of the machine of the invention.

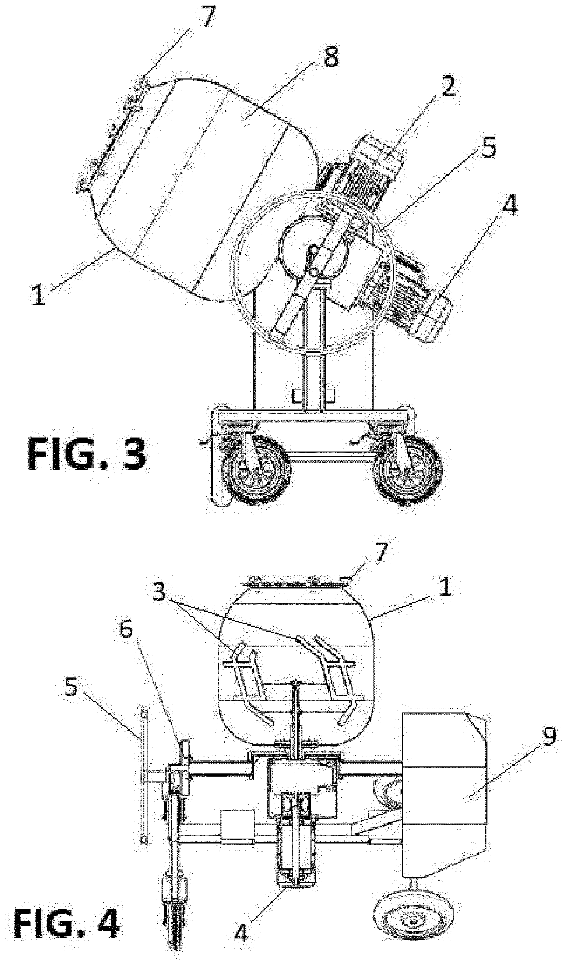

Figure 3 displays a side view of the machine of the invention.
Figure 4 displays the shredding system within the drum of the machine.

### PREFERRED EMBODIMENT OF THE INVENTION

As represented in the figures, an embodiment of the machine of the invention consists on a structure (10) with wheels, so that it can be moved by an user.

A drum (1) is fixed in the structure (10). The drum (1) is connected to a couple of motors (2, 4) and also to a handwheel (5) through a gearbox (6). The structure (10) also holds an electrical panel (9) that takes care of the energy supply of the machine.

The machine consists of several components with capacity to perform different functions effectively which are based on shredding organic waste and treating it through a biological synthesis process.

It must be noted that the machine is not exclusively linked to the aforementioned process. It can also be used for other purposes that require organic crushing. Additionally, it is designed for the digestion process of the larvae.

The machine is configured to perform different movements which, together with a thermal component, allow for adequate aeration and temperature control of the place where the organic waste is located. It is well known in the art that the larvae must digest organic waste under optimal environmental conditions.

Following is a detailed description of the components that constitute the invention. These components are categorized into the movements it performs, the resulting aeration, temperature and humidity control, energy consumption, and the structure (10) of the machine.

The machine comprises a drum (1) where the organic waste and the larvae are intended to be located. The drum (1) is connected to a first motor (2) that provides a rotational movement that allows mixing of the product that lays within. The drum (1) is provided with blades (3) configured to move the product that it contains.

A second motor (4) is connected to the blades (3), so that the blades (3) and the drum (1) move at different angular speeds. The use of the rotation of the drum (1) and the rotation of the blades (3) in combination allows the shredding of organic waste without the addition of liquids. The machine comprises as well a handwheel (5) coupled to a gearbox (6) which provides the drum (1) with an additional rotational movement that allows it to be emptied.

The machine is equipped with a mesh lid (7) that allows for proper oxygenation of the larvae while preventing them to escape. The lid (7) also provides a clear view of the production process. The lid (7) is fastened to the edge of the opening of the drum (1) so that its closure is both convenient and fast, and it can withstand stress when the drum (1) is tilted and waste rests on it.

The machine includes a heating blanket (8) that is electrically connected so that the temperature inside the drum (1) is raised to an optimal level for the larvae to digest organic waste quickly. A thermostat regulates the temperature by switching off the heating blanket (8), so that it does not exceed an appropriate level. Once it reaches this appropriate level, the larvae generate heat during the digestion process, reducing the energy required to maintain the temperature inside. No additional component has been included to control the humidity inside the drum (1), as the parameter varies significantly depending on the type of biowaste.

The thermostat may be regulated to work at temperatures between 24 and 35 °C, although these values may be settled in the machine by the user.

The power supply for the machine is a single-phase current of 230 V at 50 Hz, to supply the first motor (2), the second motor (4), the heating blanket (8) and the control system by means of a transformer and a control panel consisting of a power circuit and a control circuit in the electrical panel (9).

The machine is supported by a structure (10) resembling a concrete mixer, made up of tubular and sturdy components.

Following is a description of the working procedure of the machine of the invention.
a) With the machine stopped, open the lid (7) and load the drum (1) with organic waste.
b) Close the lid (7) and switch on the first motor (2) to rotate the drum (1).
c) Switch on the second motor (4) to rotate the blades (3). This will produce simultaneous movements of the drum (1) and blades (3) in opposite rotational directions. The drum (1) rotates at an angular speed relatively slower than the blades (3), cutting the material properly. The drum (1) is swung so that the residue that remains at the bottom of the drum (1) is raised and lowered to be cut again. The combination of the three movements homogenises and reduces the particle size of the waste. This allows the larvae to digest the shredded waste without any complications.
d) Switch off the motors (2, 4) once the machine has completed shredding the waste.
e) Open the lid (7) and introduce the larvae for the digestive process.
f) Switch on the heating blanket (8) to heat the shredded waste and larvae to an optimal temperature controlled by the thermostat for the larvae to digest the waste correctly and for biological transformation to occur.
g) Tilt and rotate the drum (1) with the handwheel (5) to aerate the content within and provide optimal oxygenation for the larvae.
h) When the larvae have finished digesting all the shredded organic waste, switch on the first motor (2) and the second motor (4) to carry out a final shredding using the blades (3) during a determined period of time.
i) Switch off the motors (2, 4) and the heating blanket (8).
j) When the machine is stopped, open the lid (7) and discharge the drum (1) by turning the handwheel (5) so that the drum (1) tilts and the product falls by its own weight into a container located at the base of the machine.

Before discharging the drum, it may be interesting to switch on the first motor and the second motor for a predetermined period of time. In this situation, the larvae are shredded and mixed with the organic waste.

Considering the described working procedure, it may be seen that the machine is suitable for processing organic waste efficiently and convert it into biomass that can be used for various purposes, including fertilizers, biofuels and phytosanitary applications.

The machine is used to rear larvae to a larger size in a shorter time than other technologies.

The machine is also suitable for other production processes that involve the movements and technical specifications mentioned above, like processing wet materials that require cutting in a dry environment without the addition of water.

The technology ensures to have organic waste in a particle size that is small enough to be processed properly by the larvae. Additionally, the machine is robust to process a considerable volume of waste and experiences minimal wear and tear when processing organic waste.

The larvae consume organic waste at a high speed, reducing the processing time and increasing the amount of waste that can be processed. As a result, the larval mass increases considerably.

The larvae do not look like mermaids; their longevity is high and they can grow considerably and uniformly. The machine maintains an optimal digestion temperature and adequate relative humidity and oxygenation levels inside the drum (1). Under these conditions, the larvae fatten quickly, considerably increasing the production of larval mass.

## Claims

1. Organic waste processing and larvae fattening machine that comprises a structure (10) which holds:
- a drum (1), configured to contain the organic waste and the larvae,
- mixing paddles located within the drum (1) for the homogenization of the product contained,
- a handwheel (5) connected to the drum (1) through a gearbox (6), to empty the contain of the drum (1),
- a first motor (2) for the rotation of the drum (1), and
- an electrical panel (9), to manage the machine,
**characterized in that:**
- the mixing paddles are blades (3), so that the contain is shredded and not only moved, the machine further comprises:
- a lid (7) to close the opening of the drum (1),
- a second motor (4) for the rotation of the blades (3),
- a heating blanket (8) located around the drum (1),
wherein
- the second motor (4) rotates at a slower angular speed than the drum (1) and in the opposite direction,
- the heating blanket (8) comprises a thermostat to regulate the temperature within the drum (1).

2. The machine of claim 1, wherein the thermostat is configured to work between 24 °C and 35 °C.

3. The machine of claim 1, wherein the drum (1) comprises a plurality of holes closed with corresponding plugs.

4. The machine of claim 1, wherein the structure (10) comprises wheels, so that it can be moved.

5. Procedure for the processing of organic waste and the fattening of larvae with the machine of claims 1-5 comprising the following steps starting with the machine off:
a) open the lid (7),
b) load the drum (1) with organic waste,
c) close the lid (7),
d) switch on the first motor (2) and the second motor (4),
e) let the machine work for a predetermined period of time,
f) switch off the motors (2, 4),
g) open the lid (7),
h) introduce the larvae inside the drum (1),
i) close the lid (7),
j) switch on the heating blanket (8),
k) let the digestion process work for a predetermined period of time,
l) switch off the heating blanket,
m) switch on the first motor for a predetermined period of time,
n) decide whether to go to step j) or o)
o) turn off the heating blanket,
p) open the lid (7),
q) turn the handwheel (5) to empty the drum (1).

6. The procedure of claim 5 which, after step o) additionally comprises the following steps:
o1) switch on the first motor (2) and the second motor (4),
o2) let the machine work for a predetermined period of time,
o3) switch off the motors (2, 4).

7. The procedure of claim 5 which comprises the step of opening the plurality of holes:
